# EUROPEAN PATENT SPECIFICATION

(11) **EP 3 413 710 B1**
(45) Date of publication and mention of the grant of the patent: **11.05.2022**
(21) Application number: 17750533.6
(22) Date of filing: 13.02.2017
(51) Int. Cl.: A01N 25/34, B82B 1/00, B82B 3/00, C23C 22/60, C23C 22/63, B82Y 5/00, B82Y 30/00, B82Y 40/00

(54) **ANTI-BACTERIAL PATTERNED SURFACES AND METHODS OF MAKING THE SAME**
ANTIBAKTERIELLE STRUKTURIERTE OBERFLÄCHEN UND VERFAHREN ZUR HERSTELLUNG DAVON
SURFACES STRUCTURÉES ANTIBACTÉRIENNES ET LEURS PROCÉDÉS DE FABRICATION

(30) Priority: 12.02.2016 SG 10201601055Y
(43) Date of publication of application: 19.12.2018
(60) Divisional application: 20170970.6
(73) Proprietor: Agency for Science, Technology and Research, Singapore 138632 (SG)
(72) Inventor: ZHANG, Yugen, Singapore 138669 (SG); YI, Guangshun, Singapore 138669 (SG)
(74) Representative: Fabry, Bernd
(86) International application number: PCT/SG2017/050063
(87) International publication number: WO 2017/138890

(56) References cited:
- WO-A1-2012/005723
- WO-A1-2014/109722
- WO-A1-2015/031956
- CN-A- 103 397 326
- CN-A- 105 018 918
- US-A- 3 544 389
- US-A- 4 702 793
- US-A- 4 844 981
- C M PELICANO ET AL: "Formation of copper oxide nanostructures by solution-phase method for antibacterial application", PROCEEDINGS OF THE INTERNATIONAL CONFERENCE ON ADVANCED MATERIALS, STRUCTURES AND MECHANICAL ENGINEERING, 1 January 2015 (2015-01-01), pages 203-206, XP055408594, Incheon, South Korea DOI: 10.1201/b19693-43
- AKHAVAN O ET AL: "Hydrothermal synthesis of ZnO nanorod arrays for photocatalytic inactivation of bacteria", JOURNAL OF PHYSICS D: APPLIED PHYSICS, INSTITUTE OF PHYSICS PUBLISHING LTD, GB, vol. 42, no. 22, 21 November 2009 (2009-11-21), page 225305, XP020166703, ISSN: 0022-3727
- MAYUREE JAISAI ET AL: "Paper modified with ZnO nanorods - antimicrobial studies", BEILSTEIN JOURNAL OF NANOTECHNOLOGY, vol. 3, 11 October 2012 (2012-10-11), pages 684-691, XP055585564, DOI: 10.3762/bjnano.3.78
- EKTHAMMATHAT NUENGRUETHAI ET AL: "Characterization and antibacterial activity of nanostructured ZnO thin films synthesized through a hydrothermal method", POWDER TECHNOLOGY - ELECTROSTATIC PHENOMENA IN PARTICULATE PROCESSES, ELSEVIER, BASEL (CH), vol. 254, 13 January 2014 (2014-01-13), pages 199-205, XP028661967, ISSN: 0032-5910, DOI: 10.1016/J.POWTEC.2014.01.010
- KRISHNA R RAGHUPATHI ET AL: "Size-Dependent Bacterial Growth Inhibition and Mechanism of Antibacterial Activity of Zinc Oxide Nanoparticles", LANGMUIR, vol. 27, no. 7, 5 April 2011 (2011-04-05), pages 4020-4028, XP055257049, US ISSN: 0743-7463, DOI: 10.1021/la104825u
- PELICANO, C. M. ET AL.: 'Formation of copper oxide nanostructures by solution-phase method for antibacterial application' PROCEEDINGS OF THE INTERNATIONAL CONFERENCE ON ADVANCED MATERIALS, STRUCTURES AND MECHANICAL ENGINEERING 29 May 2015, INCHEON, SOUTH KOREA, pages 203 - 206, XP055408594
- HU , L. ET AL.: 'Field electron emission from structure-controlled one- dimensional CuO arrays synthesized by wet chemical process' JOURNAL OF SEMICONDUCTORS vol. 35, no. 7, 31 July 2014, pages 073003 - 1 - 073003-4, XP055408598
- MAITI, S. ET AL.: 'Ambient condition oxidation of zinc foil in supersaturated solution for shape tailored ZnO nanostructures: low cost candidates for efficient electron emitter and UV-detector' CRYSTENGCOMM vol. 16, 19 November 2013, pages 1659 - 1668, XP055408599
- SIRELKHATIM, A. ET AL.: 'Review on Zinc Oxide Nanoparticles: Antibacterial Activity and Toxicity Mechanism' NANO-MICRO LETTERS vol. 7, no. 3, 19 April 2015, pages 219 - 242, XP055408600
- OKYAY, T. O. ET AL.: 'Antibacterial properties and mechanisms of toxicity of sonochemically grown ZnO nanorod' RSC ADVANCES vol. 5, 02 December 2014, pages 2568 - 2575, XP055408603

## Description

### Technical Field

The present invention generally relates to a method of killing or inhibiting the growth of a microorganism by providing a zinc substrate with anti-bacterial properties.

### Background Art

About 80% of infectious diseases caused by microorganisms are spread via contact and thus poses a serious threat to public health. Therefore, killing microorganisms on frequently touched surfaces is an effective way to avoid cross-infection.

A common method to kill microorganisms on such surfaces is by chemical means, such as disinfectants. In another method, antimicrobial surfaces are fabricated by grafting or coating the surfaces with biocidal chemicals or disinfectants to limit cross-infections. However, microorganisms may evolve and develop resistance to the current biocidal chemicals and new chemicals would then need to be developed. Killing via chemical means therefore contributes to secondary contamination. Hence, these methods face challenges such as growing drug resistance to the microbicide agents, low microbial killing efficacy and poor long term stability of coated surfaces.

It was recently discovered that cicada and dragonfly wing surfaces are covered with dense pillared nanostructures that kill microbes or prevent microbial growth by rupturing adhered microbial cells due to a purely physical interaction between the wing surfaces and the microbial cells. The interaction results in cell deformation and lysis without the need for additional external chemical or mechanical means. However, there are presently no known methods that can provide nano-arrayed surfaces capable of physical cell destruction by mimicry of biological surfaces in an efficient and simple manner.

Nanostructures on surfaces of black silicon and TiO₂ have demonstrated microbicidal properties. However, these surface nano-patterns were generated by a top-down approach on specific materials. For example, the black silicon surface was prepared by reactive-ion beam etching on a silicon wafer. Thus, it may be appreciated that the top-down approach would become challenging when nanometer scale patterns are to be generated. In other words, top-down approaches can be time-consuming and expensive and are limited to application on surfaces of specific materials (e.g., those susceptible to etching or other forms of lithographic methods).

In C M PELICANO ET AL: "Formation of copper oxide nanostructures by solution-phase method for antibacterial application", published in PROCEEDINGS OF THE INTERNATIONAL CONFERENCE ON ADVANCED MATERIALS, STRUCTURES AND MECHANICAL ENGINEERING on January 1, 2015 (XP055408594) it is described a method of killing or inhibiting the growth of a microorganism by contacting the microorganism with a copper substrate having specific nanostructures grown thereon.

The patent application WO 2014/109722 A1 discloses a reagent solution comprising 10 M of copper nitrate trihydrate as oxidizing agent for synthesis of CuO nanotubes, in particular to inhibit the growth of *Staphylococcus aureus.*

AKHAVAN O ET AL: "Hydrothermal synthesis of ZnO nanorod arrays for photocatalytic inactivation of bacteria", published in JOURNAL OF PHYSICS, INSTITUE OF PHYSICY PUBLISHING LTD, GB on November 21, 2009 (XP020166703) and MAYUREE JAISAI ET AL:" Paper modified with ZnO nanorods - antimicrobial studies", published in BEILSTEIN JOURNAL OF NANOTECHNOLOGY on October 11, 2012 (XP055585564) disclose a method of killing or inhibiting the growth of microorganisms by contacting the microorganisms with a ZnO seed layer substrate having ZnO nanorods grown thereon.

Ekthammathat Nuengruethai et al, "Characterization and Antibacterial Activity of Nanostructured ZnO Thin Films Synthesized Through a Hydrothermal Method", Powder Technology-Electrostatic Phenomena in Particulate Processes, 254, 13 January 2014, pages 199-205 disclose the preparation of nanostructured ZnO films. The ZnO nanostructures are prepared by exposing a zinc foil to alkaline precursor solutions and subsequently autoclaving the mixture at 120°C for 24 hours. Structures such as rod, pencils and stars may be formed on the zinc foil using NaOH, LiOH and NH₄OH.

Krishna R Raghupathi et al, "Size-dependent Bacterial Growth Inhibition and Mechanism of Antibacterial Activity of Zinc Oxide Nanoparticles", Langmuir, 27(7), 5 April 2011, pages 4020-4028 disclose the antibacterial properties of ZnO nanoparticles against microorganisms such as Staphylococcus aureus. The antibacterial properties of the ZnO nanoparticles against such microorganisms was inversely proportional to the size of the nanoparticles. The antibacterial properties of the nanoparticles is suggested to be related to the generation of reactive oxygen species and the accumulation of the nanoparticles within the cytoplasm or outer membrane of the microorganism.

Hence, there is a need to provide alternative surfaces demonstrating microbicide properties that overcomes, or at least ameliorates, one or more of the disadvantages described above. There is also a need to provide simple and scalable methods to create such surfaces.

### Summary of Invention

There is provided a method of killing or inhibiting the growth of a microorganism, said method comprising the steps of: providing a zinc substrate having a plurality of, ordered, integrally-formed surface structures prepared by: contacting a surface of the zinc substrate with a reagent solution, the reagent solution comprising an alkali and a zinc nitrate; wherein the concentration of the alkali in the reagent solution is from about 1.0 M to about 2.5 M; contacting said microorganism with said zinc substrate, wherein the surface features are micro-sized, nano-sized or a mixture thereof, each surface feature comprising a crystalline phase and at least one pointed terminus.

In another aspect, there is provided the use of the zinc substrate as disclosed herein for providing antibacterial properties to an ex-vivo environment.

### Definitions

The following words and terms used herein shall have the meaning indicated:
The term "microbe" refers to one or a plurality of microorganisms which include bacteria, fungi, algae, yeasts, molds and viruses.

The term "antimicrobial" refers to anything that kills or inhibits the growth of microbes. The term "antimicrobial" can be used to describe a thing or a characteristic of the thing and in this context, refers to the ability to kill or inhibit the growth of microbes. Accordingly, the term "antibacterial" refers to anything that kills or inhibits the growth of bacteria or, when describing a thing or a characteristic of the thing, refers to the ability to kill or inhibit the growth of bacteria. The terms "antimicrobial", "microbicide" and "biocide" are used interchangeably.

The prefix "nano" denotes average sizes of a scale below 1 µm. Accordingly, the term "nano-sized", as used in the context of the specification, refers to a feature having at least one dimension, e.g. length or height, with a nanoscale size. The term "nanostructure" or grammatical variants thereof is to be interpreted accordingly, to refer to a feature or pattern, e.g. blade or tube, having at least one dimension in the nanoscale.

The prefix "micro" denotes average sizes of a scale between about 1 µm to about 1000 µm. Accordingly, the term "micro-sized", as used in the context of the specification, refers to a feature having at least one dimension, e.g. length or height, with a microscale size.

The term "crystalline" or "crystalline phase" as used herein is to be broadly interpreted to refer to a physical state having regularly repeating arrangement of molecules which are maintained over a long range or regularly repeating external face planes. The regularly repeating building blocks are arranged according to well-defined symmetries into unit cells that are repeated.in three-dimensions.

The word "substantially" does not exclude "completely" e.g. a composition which is "substantially free" from Y may be completely free from Y. Where necessary, the word "substantially" may be omitted from the definition of the invention.

Unless specified otherwise, the terms "comprising" and "comprise", and grammatical variants thereof, are intended to represent "open" or "inclusive" language such that they include recited elements but also permit inclusion of additional, unrecited elements.

As used herein, the term "about", in the context of concentrations of components of the formulations, typically means +/- 5% of the stated value, more typically +/- 4% of the stated value, more typically +/- 3% of the stated value, more typically, +/- 2% of the stated value, even more typically +/- 1% of the stated value, and even more typically +/- 0.5% of the stated value.

Throughout this disclosure, certain embodiments may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

### Detailed Disclosure of Embodiments

It is provided a zinc substrate comprising a plurality of integrally formed surface features, said surface features being micro-sized and/or nano-sized, each surface feature comprising a crystalline phase and at least one pointed terminus.

The substrate may be capable of supporting the growth of the plurality of surface features on its surface. The substrate may be capable of supporting the growth of salts of the substrate on its surface. Advantageously, the surface structures may be integrally formed by precipitation of the salts on the substrate.

The substrate may be capable of reaction to thereby integrally form the plurality of surface features on its surface. The surface of the substrate may be reactive with an oxidizing agent to thereby integrally form the plurality of surface features.

The substrate surface may be coated with one or more layers of reactive or oxidative solution to thereby integrally form the plurality of surface features on its surface. The oxidation of the substrate surface may form salts or salt crystals which are insoluble in typical organic or inorganic solvents or aqueous mediums that contact the surfaces. Hence, the crystalline phase of the surface feature may comprise the insoluble salt formed from the oxidation of the surface. The substrate surface may be coated with one or more layers of reagent solution comprising ions of salts which are insoluble in typical organic or inorganic solvents or aqueous mediums that contact the surfaces. Hence, the crystalline phase of the surface feature may comprise the insoluble salt formed by precipitation or deposition onto the substrate surface. For example, the salt or salt crystals may be insoluble in rain water, fruit juices or perspiration. Therefore, the disclosed substrate may advantageously be weather resistant and the antimicrobial and antibacterial properties of the disclosed substrate may be long-lasting. The formed surface features are advantageously ordered and crystalline, which otherwise would be difficult to obtain with top-down surface modification techniques.

The crystalline phase of the surface feature may comprise an oxide salt or a hydroxide salt. Advantageously, the oxide and hydroxide surface features may be formed *in-situ* via oxidation reactions, acid/base reactions or salt precipitation reactions. Advantageously, the fabrication of such surface features does not require complex techniques, e.g., plasma etching, reactive ion etching, physical or chemical vapor deposition techniques or lithography techniques. The oxide and/or hydroxide features may be formed via a one-pot or one-step reaction synthesis. The oxide or hydroxide surface features may advantageously be formed of a simple oxidation or precipitation reaction.

Accordingly, there is provided a substrate comprising a plurality of integrally formed surface features, wherein the surface features are micro-sized and/or nano-sized, each surface feature comprising a crystalline phase and at least one pointed terminus, and wherein the surface features are formed by, or obtainable from, a one-step process comprising contacting a surface of said substrate with an oxidizing solution comprising an alkali and an oxidizing agent to thereby integrally form the surface features on the surface of said substrate. It is postulated that the process of contacting the surface of the substrate with an oxidizing solution comprising an alkali and an oxidizing agent results in the formation of the plurality of surface features, each comprising at least one pointed terminus. Due to the nature of their chemical formation, the exact characterization of the structure of each surface feature formed from the process may not be exhaustively described by physical characteristics.

Accordingly, there is provided a substrate comprising a plurality of integrally formed surface features, wherein the surface features are micro-sized and/or nano-sized, each surface feature comprising a crystalline phase and at least one pointed terminus, and wherein the surface features are formed by, or obtainable from, a one-step process comprising contacting a surface of said substrate with a reagent solution comprising ions of salts to thereby integrally form the surface features by precipitation on the surface of said substrate. It is postulated that the process of contacting the surface of the substrate with a reagent solution comprising ions of salts results in the formation of the plurality of surface features, each comprising at least one pointed terminus, deposited or precipitated on the substrate surface. Due to the nature of their chemical formation, the exact characterization of the structure of each surface feature formed from the process may not be exhaustively described by physical characteristics.

Each surface feature comprises at least one pointed terminus. The terminus or distal end of the integrally formed surface feature is an end opposite the substrate, facing away from the substrate. Upon physical contact with microbial cells, the pointed terminus or protrusion is advantageously effective in rupturing the cell walls and thereby killing or at least inhibiting the growth of the cells. Accordingly, any microbe transferred to or contacting the disclosed substrate may advantageously be killed or inhibited from growing. Thus, the spread of infectious diseases caused by microbes may advantageously be stopped or at least slowed down.

The surface feature comprises a crystalline phase that provides the at least one pointed terminus. The crystalline phase may be selected from an orthorhombic crystal structure, monoclinic crystal structure, triclinic crystal structure, tetragonal crystal structure, hexagonal crystal structure, trigonal crystal structure or cubic crystal structure. In an embodiment, the crystalline phase has a structure selected from an orthorhombic crystal structure, monoclinic crystal structure or a hexagonal crystal structure. An example of a hexagonal crystal system is a wurtzite crystal structure. An example of an orthorhombic crystal structure is one having an X-Ray Diffraction characterization of JCPDS no. 13-0420. An example of a monoclinic crystal structure is one having an X-Ray Diffraction characterization of JCPDS no. 48-1548.

The surface feature is of a shape that provides the at least one pointed terminus. The integrally formed surface feature may be tapered in shape, having a base end coupled to a surface of the substrate and a distal end that is smaller in dimension relative to the base end. For example, the surface feature may have a shape selected from the group consisting of tubes, blades, needles, pyramids, cones, pillars and mixtures thereof. Hence, the distal end of the surface feature may refer to a tapered tip, a bladed end, a conical apex, or a pyramidal vertex. The distal end refers to a pointed terminus of a surface feature.

In an embodiment, the surface feature is a nanotube or a needle. The nanotube or needle may be tapered or may comprise a distal end having a smaller cross-sectional diameter compared to a cross-sectional diameter of its base section. The corresponding distal end may be of circular cross-section having a diameter. In another embodiment, the surface feature is a blade and the corresponding distal end may be of a rectangular cross-section having a breadth or thickness.

Exemplary dimensions of the surface features may be provided as follows.

The dimensions of the surface feature are in the micro-size scale or in the nano-size scale or a mixture of micro-size and nano-size scales. The dimensions of the surface features may be advantageously tailored according to, for example, the application of the substrate or the size of the microbe(s) intended for killing or inhibition.

The ratio of the height of the surface feature to a dimension of the terminus distal end of the surface feature may be about 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190 or 200. The ratio of the height of the surface feature to a dimension (e.g. diameter or thickness) of the terminus distal end of the surface feature may be in a range comprising an upper and lower limit selected from any two of the above values.

The height or length of the surface feature refers to a dimension from the base of the surface feature formed at the substrate surface to the distal end or terminus of the surface feature. In the context of the present disclosure, the higher the ratio of surface feature height to a dimension of the distal end of the surface feature, the sharper would be the distal end of the surface feature. A higher ratio of surface feature height to a dimension of the distal end of the surface feature signifies a higher sharpness of the distal end of the surface feature. Advantageously, it has been discovered that the sharpness of the distal end of the surface feature is proportional to the antimicrobial efficiency of the substrate. That is, the sharper the pointed terminus, the more effective the surface feature would be in killing or inhibiting the growth of the cells. Advantageously, the disclosed substrate is capable of reducing an amount of bacteria contacting the substrate to 0.5 or less of the initial CFU value per unit volume. The surface features of the disclosed substrate may have a sharpness higher than known natural or artificial biocidal surfaces. Thus, the antimicrobial efficiency of the disclosed substrate may be higher than the known biocidal surfaces.

The surface feature may possess a height selected from about 200 nm, 300 nm, 400 nm, 500 nm, 600 nm, 700 nm, 800 nm, 900 nm, 1 µm, 1.25 µm, 1.5 µm, 1.75 µm, 2 µm, 2.25 µm, 2.5 µm, 2.75 µm, 3 µm, 3.25 µm, 3.5 µm, 3.75 µm, 4 µm, 4.25 µm, 4.5 µm, 4.75 µm, 5 µm, 5.25 µm, 5.5 µm, 5.75 µm, 6 µm, 6.25 µm, 6.5 µm, 6.75 µm, 7 µm, 7.25 µm, 7.5 µm, 7.75 µm, 8 µm, 8.25 µm, 8.5 µm, 8.75 µm, 9 µm, 9.25 µm, 9.5 µm, 9.75 µm or 10 µm. The surface feature may possess a height in a range comprising an upper limit and a lower limit selected from any two of the above values.

In embodiments, the dimension of the distal end may refer to a cross-sectional diameter, a width, or a thickness of the distal end. In an embodiment, the dimension of the distal end of the surface feature is selected from diameter or thickness. The dimension of the terminus distal end of the surface feature, that is in an embodiment the diameter or thickness of the terminus distal end, is selected from about 1 nm to about 500 nm, or about 5 nm, 10 nm, 15 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, 150 nm, 155 nm, 160 nm, 165 nm, 170 nm, 175 nm, 180 nm, 185 nm, 190 nm, 195 nm, 200 nm, 205 nm, 210 nm, 215 nm, 220 nm, 225 nm, 230 nm, 235 nm, 240 nm, 245 nm, 250 nm, 255 nm, 260 nm, 265 nm, 270 nm, 275 nm, 280 nm, 285 nm, 290 nm, 295 nm, 300 nm, 305 nm, 310 nm, 315 nm, 320 nm, 325 nm, 330 nm, 335 nm, 340 nm, 345 nm, 350 nm, 355 nm, 360 nm, 365 nm, 370 nm, 375 nm, 380 nm, 385 nm, 390 nm, 395 nm, 400 nm, 405 nm, 410 nm, 415 nm, 420 nm, 425 nm, 430 nm, 435 nm, 440 nm, 445 nm, 450 nm, 455 nm, 460 nm, 465 nm, 470 nm, 475 nm, 480 nm, 485 nm, 490 nm, 495 nm or 500 nm. The dimension of the terminus distal end of the surface feature may be in a range comprising an upper limit and a lower limit selected from any two of the above values. The dimension of the distal end may advantageously be nano-sized. Advantageously, it has been shown that substrates with surface features having tapered ends of between about 10 nm and 400 nm, about 10 nm and 300 nm or about 10 nm and 200 nm in dimension are capable of killing between 90 - 100% of the bacteria *S. aureus* after just one hour of incubation. Further advantageously, the resolution (and size) of the surface features of the present disclosure are not limited by the resolution provided by conventional surface modification techniques. Even further advantageously, the *in-situ* formation of surface features via chemical reaction allows the formation of surface features having terminal dimensions as small as 10 nm.

In an example, when the surface feature is a tube, the height of the tube may range from about 1 µm to 10 µm or about 5 µm to 7 µm; and the distal end may be a tip of circular cross-section having a diameter of from about 50 nm to 300 nm or about 100 nm to 200 nm.

In another example, when the surface feature is a blade, the blade may have a length of from about 200 nm to 5 µm or about 400 nm to 1 µm; and a breadth of from about 100 nm to 500 nm or about 200 nm to 400 nm. The thickness of the blade may be tapered towards the distal end of the blade. The distal end of the blade may be a bladed end having a thickness of from about 10 nm to 30 nm, or about 20 nm.

In yet another example, when the surface feature is a needle, the length of the needle may range from about 500 nm to 5 µm or about 1 µm to 2 µm; the distal end may be a tip of circular cross-section having a diameter of about 1 nm to 100 nm or about 10 nm to 40 nm; and the base or root of the needle may be of circular cross-section having a diameter of about 10 nm to 500 nm or about 100 nm to 200 nm.

The pitch of adjacent surface features may be selected from about 100 nm, 200 nm, 300 nm, 400 nm, 500 nm, 600 nm, 700 nm, 800 nm, 900 nm, 1000 nm, 1100 nm, 1200 nm, 1300 nm, 1400 nm, 1500 nm, 1600 nm, 1700 nm, 1800 nm, 1900 nm or 2000 nm. The pitch of adjacent surface features may be in a range comprising an upper limit and a lower limit selected from any two of the above values.

As microbial and bacterial cells are typically larger than the disclosed pitch, surface features with the disclosed pitch are advantageously capable of contacting and rupturing the cells, thereby conferring antimicrobial and antibacterial properties on the substrate.

It is provided a substrate comprising a zinc surface, said zinc surface comprising a plurality of micro-sized and/or nano-sized ZnO surface features integrally formed thereon, said ZnO surface features comprising at least one pointed terminus.

Advantageously, zinc is a surface material commonly encountered in daily life, e.g. street lamp poles and highway guardrails comprise galvanized steel with Zn surfaces. Hence, it is an advantage that the present disclosure can be applied to common surfaces, such as zinc surfaces, to provide microbicidal surfaces effective in killing or at least inhibiting the growth of microbes via physical means or physical interaction.

Zinc substrates have been advantageously found to provide ease of fabricating the disclosed surface features using straightforward synthesis steps. The use of zinc substrates avoids the need for top-down texturing techniques, e.g., reactive-ion beam etching commonly employed on silicon based substrates. The resolution and size of the surface features of the present disclosure are also advantageously not limited by the resolution provided by conventional surface modification techniques.

Exemplary, non-limiting embodiments of a method of producing a substrate possessing antimicrobial or antibacterial properties will now be disclosed.

It is provided a method of producing a substrate possessing antimicrobial or antibacterial properties, the method comprising: contacting a surface of the zinc substrate with a reagent solution to produce a plurality of integrally formed, micro-sized or nano-sized surface features on the substrate surface, each surface feature comprising a crystalline phase and at least one pointed terminus.

In embodiments, there is provided a method of producing a substrate possessing antimicrobial or antibacterial properties, the method comprising: contacting a surface of the zinc substrate with a reagent solution to produce a plurality of integrally formed, micro-sized or nano-sized surface features by precipitation on the substrate surface, each surface feature comprising a crystalline phase and at least one pointed terminus.

Exemplary reaction at the surface of zinc substrate is shown below:

Zn²⁺ + 4OH⁻ → Zn(OH)₄²⁻+ NO₃⁻ → ZnO

The reagent solution may comprise an oxidizing agent selected from halogens, oxygen, peroxides, hypohalites, chlorates, chromates, persulfates, permanganates, nitrates or nitric acid. Examples include ammonium persulfate, zinc nitrate, hydrogen peroxide and sodium hypochlorite.

The concentration of the oxidizing agent in the reagent solution may be selected from about 0.01 M to about 10 M, or 0.02 M, 0.04 M, 0.06 M, 0.08 M, 0.1 M, 0.12 M, 0.14 M, 0.15 M, 0.16 M, 0.17 M, 0.18 M, 0.19 M, 0.2 M, 0.3 M, 0.4 M, 0.5 M, 1.0 M, 1.5 M, 2.0 M, 2.5 M, 3.0 M, 3.5 M, 4.0 M, 4.5 M, or 5.0 M. The concentration of the oxidizing agent in the reagent solution may be in a range comprising an upper limit and a lower limit selected from any two of the above values. Advantageously, the concentration of the oxidizing agent may be suitably selected to provide specific surface feature dimensions as required by the application of the produced metal substrate. A higher concentration of the oxidizing agent may be selected to result in surface features comprising a monoclinic crystal structure, while a lower concentration of the oxidizing agent may be selected to result in surface features comprising an orthorhombic crystal structure. For example, where the concentration of the oxidizing agent is at least about 0.3 M, surface features comprising a monoclinic crystal structure may be obtained.

The reagent solution comprises an Examples include NaOH and KOH. The concentration of the alkali in the reagent solution is from 1.0 M to 2.5 M, preferably, 1.5 M or 2.0 M. concentration of the alkali in the reagent solution may be in a range comprising an upper limit and a lower limit selected from any two of the above values. Advantageously, the concentration of the alkali may be suitably selected to provide specific surface feature dimensions as required by the application of the produced metal substrate. In embodiments, a higher concentration of the alkali may be selected to result in surface features comprising a monoclinic crystal structure, while a lower concentration of the alkali may be selected to result in surface features comprising an orthorhombic crystal structure.

The reagent solution may further comprise water. The concentration of the reagent solution may be adjusted by addition of water.

The reagent solution comprises zinc nitrate and an alkali as disclosed herein, such as KOH, wherein the zinc ion and the hydroxide ion ultimately results in the insoluble zinc oxide salt precipitated on the substrate surface. The reagent solution may not comprise an oxidizing agent.

The contacting step may be conducted for a duration sufficient to produce the plurality of surface features. The duration may be suitably selected to provide specific surface feature dimensions as required by the application of the produced metal substrate. In an example, where the substrate is zinc and surface features comprising a wurtzite crystal structure are desired, the contacting step may be conducted for a duration of about 6 to 18 hours.

Accordingly, the concentration of the alkali, or the concentration of the oxidizing agent, or the concentration of the ions, or the duration of the contacting step, or the temperature of the contacting step, or any combination thereof, may be selected to provide specific surface feature dimensions as required by the application of the produced metal substrate. An increase in the concentration of the alkali, or an increase in the concentration of the concentration of the oxidizing agent, or an increase in both the concentrations of the alkali and the oxidizing agent may result in surface features comprising a monoclinic crystal structure.

The contacting step may be conducted at room temperature or ambient temperature, or about 15°C, or about 20°C, or about 25°C, or about 30°C. Advantageously, the disclosed method is capable of being conducted without the use of specialized equipment, such as pressurized chambers or heat-rated vessels.

The substrate may be transformed into a substrate possessing antimicrobial/antibacterial properties using the disclosed one-step method. In one embodiment, the surface features may be formed via a one-pot or one-step reaction synthesis. Thus, the disclosed surface features may be formed *in-situ* via simple oxidation reactions or acid/base reactions or precipitation reactions. The disclosed method is therefore advantageous and cost-effective over known complex techniques of fabricating surface features on metal substrates. Advantageously, the disclosed method is capable of providing the surface features without being limited by the resolution of a mold as required by conventional etching or lithography techniques. Advantageously, the disclosed method does not require complex or multi-step nano-imprinting or screen printing methods to obtain nano-sized surface features on the surface of the substrate. Advantageously, fabrication of the disclosed surface features does not require complex techniques, e.g., plasma etching, reactive ion etching, physical or chemical vapor deposition techniques. Advantageously, the disclosed method can be used with "hard" metal substrates that may not be malleable to conventional surface modification techniques. Advantageously, the disclosed method is capable of preparing metal substrates capable of bio-mimicry, e.g., replicating or simulating physical, non-chemical microbe/bacteria-killing properties found in nature.

The present disclosure provides the use of a zinc substrate as disclosed herein for providing antimicrobial and antibacterial properties to an ex-vivo environment. The disclosed substrate may provide bacteriostatic or bactericidal purposes to the ex-vivo environment. Accordingly, as the use of the substrate is in an ex-vivo environment, the use may be a non-therapeutic one.

Alternatively, the disclosed substrate may be used in therapy. The disclosed substrate may be used in the treatment of microbial infections.

The disclosed substrate may be capable of killing or inhibiting the growth of microbes. The microbes may be pathogenic or non-pathogenic. The microbes may be bacteria or fungi. The bacteria may include gram-negative and gram-positive bacteria.

Examples of gram-positive bacteria include Staphylococcus, Enterococcus and Streptococcus, such as *Staphylococcus aureus*, *Enterococcus faecalis*, *Bacillus megaterium*, *Hay bacillus*, *Mycobacterium smegmatis* and *Streptococcus pneumoniae.* Examples of gram-negative bacteria include Escherichia, Shigella and Salmonella, such as *Escherichia coli*, *Pseudomonas aeruginosa*, *Chlamydia trachomatis*, *Helicobacter pylori*, *Shigella dysenteriae*, *Salmonella enteritidis* and

*Salmonella typhi.*

### Brief Description of Drawings

The accompanying drawings illustrate a disclosed embodiment and serves to explain the principles of the disclosed embodiment. It is to be understood, however, that the drawings are designed for purposes of illustration only, and not as a definition of the limits of the invention.

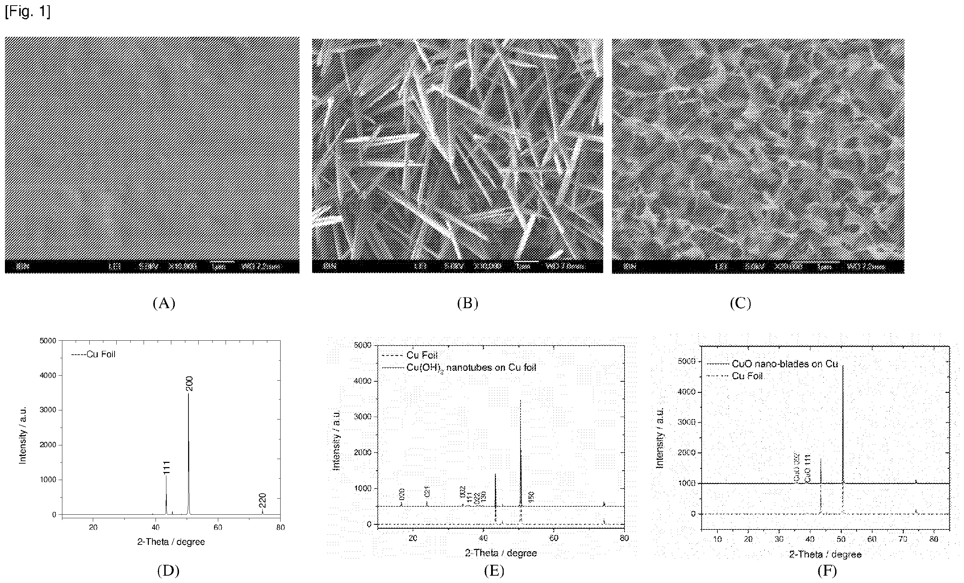

**Fig. 1** (not according to the invention)
   [Fig. 1] contains the Scanning Electron Microscopy (SEM) images of (A) Cu foil, (B) Cu(OH)2 nanotubes growing on Cu foil, (C) CuO nano-blades growing on Cu foil and the graphs of their corresponding X-Ray Diffraction (XRD) patterns (D-F), confirming their respective structures.
**Fig. 2**
   [Fig. 2] contains the SEM images of (A) Zn foil, (B, C) ZnO nano-needles growing on Zn foil, and (D) graph of the XRD pattern of ZnO nano-needles on Zn foil.
**Fig. 3** (not according to the invention)

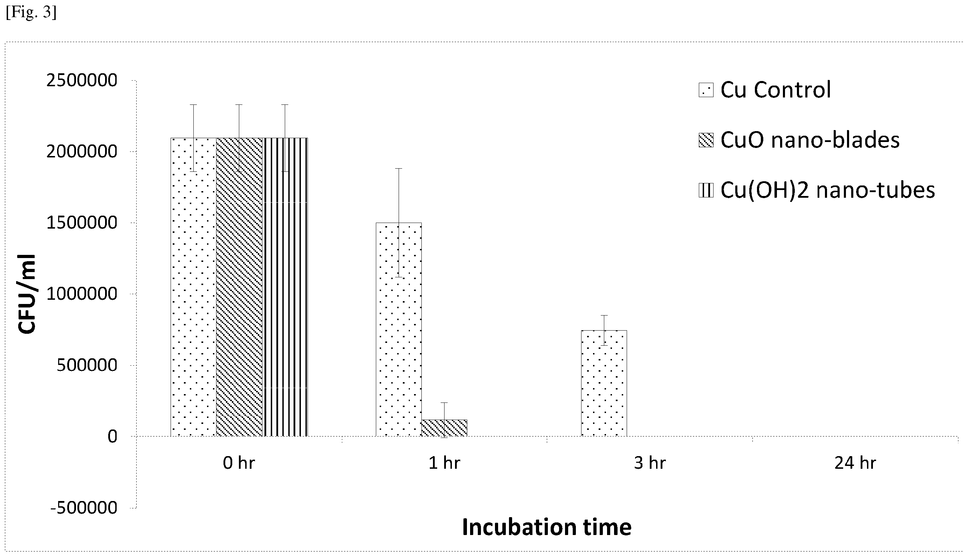

   [Fig. 3] is a graph of the Colony Forming Units (CFU)/ml against the incubation time showing the killing efficacy (against *E. coli*) of various copper surfaces evaluated using Japanese Industrial Standard (JIS) Z 2801/ISO 22196 method.

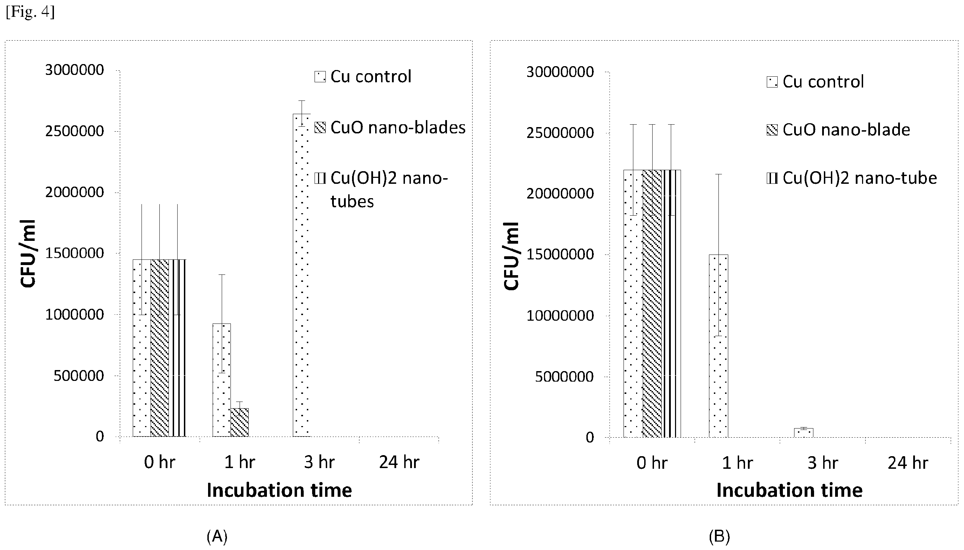

**Fig. 4** (not according to the invention)
   [Fig. 4] contains graphs of the CFU/ml against the incubation time demonstrating the killing efficacy (against *E. coli*) of various copper surfaces evaluated using JIS Z 2801/ISO 22196 method for (A) samples with Pt coating and (B) samples with Cu coating.
**Fig. 5**

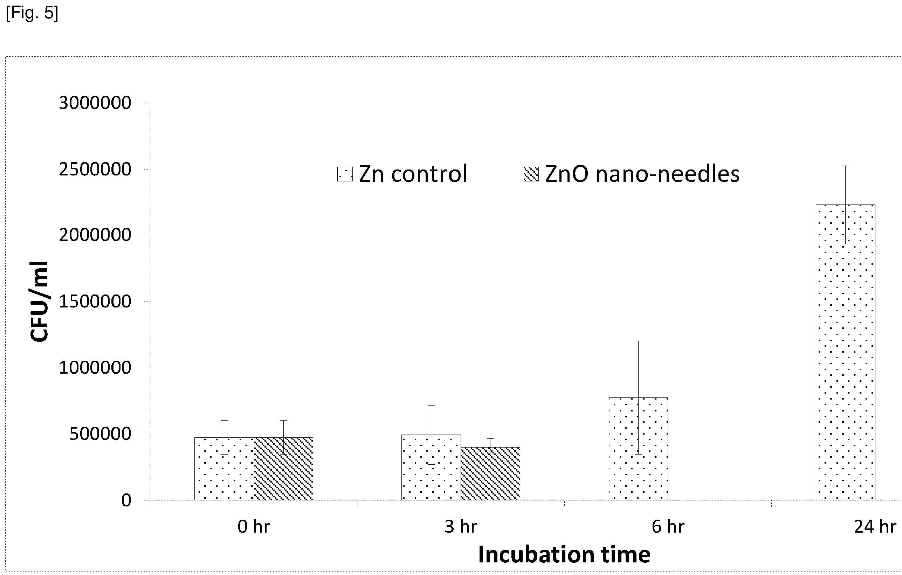

   [Fig. 5] is a graph of the CFU/ml against the incubation time showing the killing efficacy (against *E. coli*) of flat Zn foil and ZnO nano-needle surface evaluated using JIS Z 2801/ISO 22196 method.

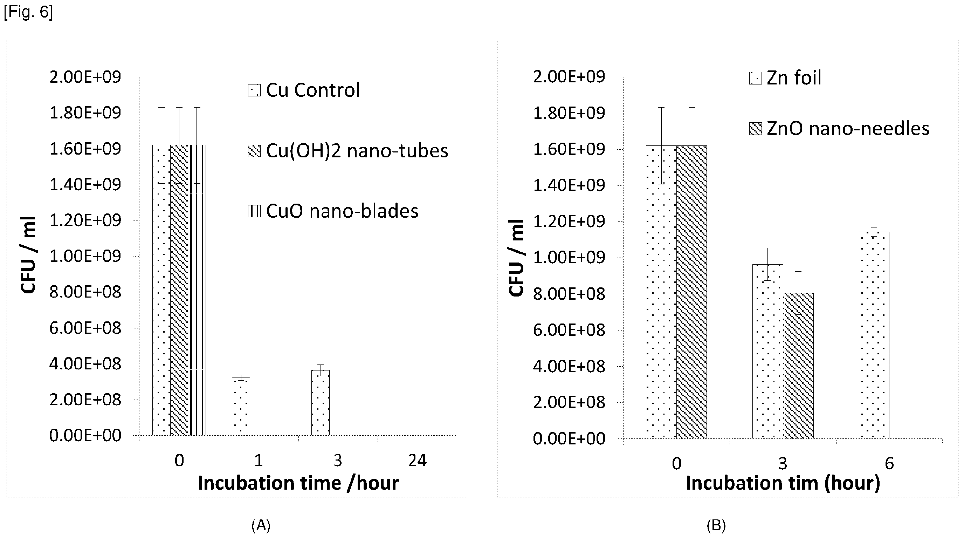

**Fig. 6** (figure 6(A) not according to the invention)
   [Fig. 6] contains graphs of the CFU/ml against the incubation time demonstrating the killing efficacy (against *S. aureus*) of (A) flat Cu foil, Cu(OH)2 nano-tubes, CuO nano-blades surface, and (B) flat Zn foil and ZnO nano-needle surface evaluated using JIS Z 2801/ISO 22196 method.

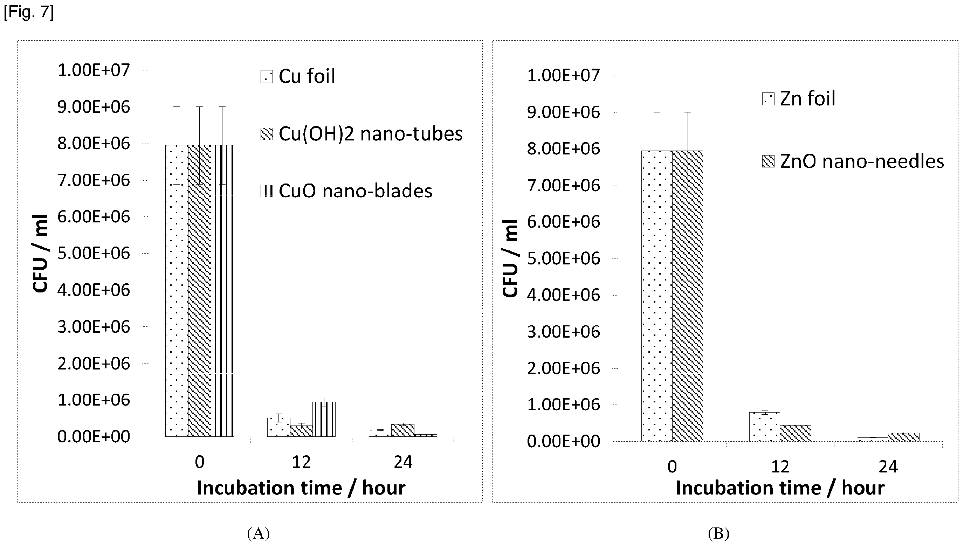

**Fig. 7** (figure 7(A) not according to the invention)
   [Fig. 7] contains graphs of the CFU/ml against the incubation time demonstrating the killing efficacy (against *C. albicans*) of (A) flat Cu foil, Cu(OH)2 nano-tubes, CuO nano-blades surface, and (B) flat Zn foil and ZnO nano-needle surface evaluated using JIS Z 2801/ISO 22196 method.

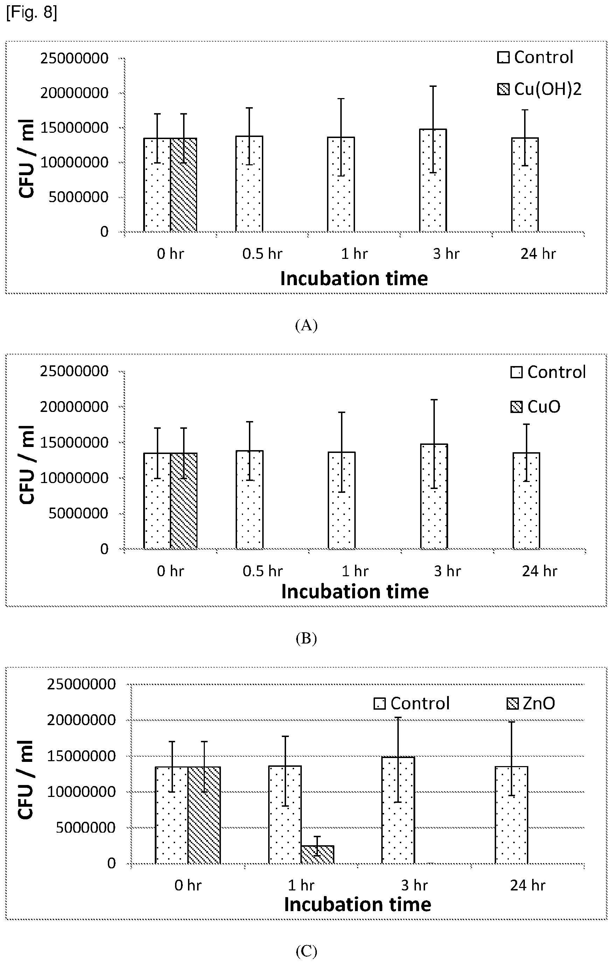

**Fig. 8** (figures 8(A) and 8(B) not according to the invention)
   [Fig. 8] contains graphs of the CFU/ml against the incubation time demonstrating the killing profiles (against *E. coli*) of nano-structured surfaces (A) Cu(OH)2 nanotubes surface, (B) CuO nano-blades surface, and (C) ZnO nano-needles surface in water under shaking condition. Testing conditions: 5 ml water, 37 °C, shaking at 300 r/min.

### Examples

Non-limiting examples of the invention and a comparative example will be further described in greater detail by reference to specific Examples, which should not be construed as in any way limiting the scope of the invention.

### Example 1: Preparation of Cu(OH)₂ nanotubes and CuO nanoblades on Cu substrate (not according to the invention)

For the growing of Cu(OH)2 nanotubes, 4 ml of 1M (NH₄)₂S₂O₈, 8 ml of 10M NaOH and 18 ml of water were mixed to form a solution. A Cu foil (20 x 25 mm) was suspended in the solution for 15 min. A solid film of Cu(OH)2 nanotubes was obtained on the Cu foil. The Cu foil was then washed 3 times with water and 3 times with ethanol. After washing, the foil was dried with flowing N2 and stored for future use.

For the growing of CuO nanoblades, 4 ml 1M (NH₄)₂S₂O₈ solution and 8 ml 10M NaOH were mixed. A Cu foil (20 x 25 mm) was suspended in the solution for 30 min. A black solid film of CuO nanoblades was obtained on the Cu foil. The Cu foil was then washed 3 times with water and 3 times with ethanol. After washing, the foil was dried with flowing N2 and stored for future use.

### Example 2: Preparation of ZnO nanoneedles on Zn substrate

For the growing of ZnO nanoneedles, 10 ml of 0.5M Zn(NO₃)₂ aqueous solution and 10 ml 4M KOH were mixed. A Zn foil (20 x 20 mm) was suspended in the solution for 12 h at room temperature. The surface of the Zn foil was washed 3 times with water and 3 times with ethanol. Subsequently, the Zn foil was dried with flowing N2 and stored for future use.

### Example 3: Characterization of surface

The surfaces of the samples were characterized by SEM (JEOL JSM-7400E) and XRD (PANalytical X-ray diffractometer, X'pert PRO, with Cu Kα radiation at 1.5406 A). Prior to SEM, the samples were coated with thin Pt film using high resolution sputter coater (JEOL, JFC-1600 Auto Fine Coater). Coating conditions: For sample testing (20 mA, 30 s). For Pt coated sample for antibacterial testing (20 mA, 60 s).

Nano-patterns on copper substrate was prepared by treatment of copper foil in a (NH₄)₂S₂O₈ and NaOH solution at room temperature (see Example 1, which is not according to the invention), 2 types of nano-structures were grown on copper substrate. As shown in Fig. 1, when copper foil was treated with lower concentration of the solution for 15 min, nanotubes array was grown. The nanotube array grew upwards and covered the whole area of the copper substrate compactly. Each tube was 5-7 µm in length with an open and sharp tip of ~100-200 nm diameter. XRD confirmed the structure was Cu(OH)₂ with orthorhombic phase (JCPDS Card No. 13-0420). When the cupper foil was treated with higher concentration of the solution at ambient temperature, blade-like structure was formed on the Cu surface, with sharp edge standing upward. XRD confirmed the structure to be monoclinic symmetry of CuO on copper. (JCPDS Card No. 48-1548).

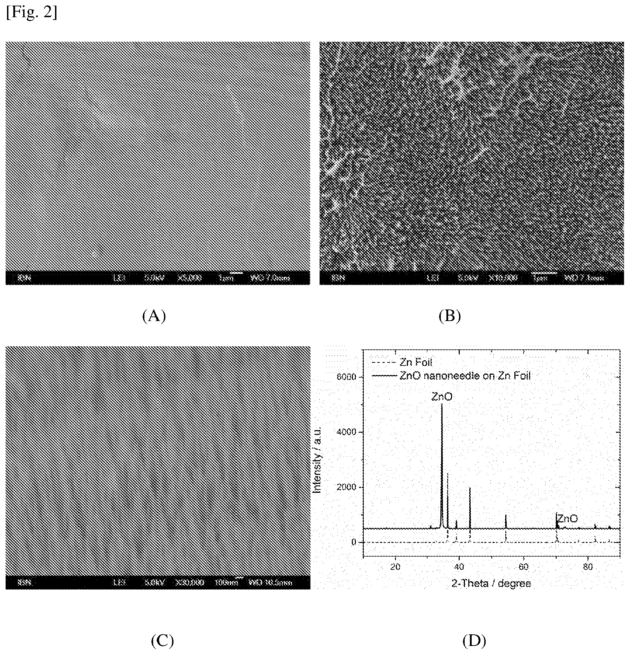

Similarly, a nano-patterned zinc surface was prepared by using a simple method (see Example 2). By treatment of a zinc foil in Zn(NO₃)₂ and KOH solution, ZnO nano-needles array was grown on the zinc substrate as shown below in Fig. 2. After treatment in the solution for 12 hours at room temperature, highly oriented uniform nano-needles array was formed on the surface. Further study showed that the needles were typically 1-2 µm in length. The diameters of the needle tips and roots are 10-40 nm and 100-200 nm, respectively. XRD analysis confirmed that the nano-needles are wurtzite ZnO structure. A strong diffraction peak at 34.4 ° (002) was present, indicating the highly preferential growth of ZnO nanoneedles along c-axis.

### Example 4: Bacterial growth conditions and sample preparation

*E. coli, S. aureus, and C. albicans* were obtained from American Type Culture Collection (ATCC-8739). Prior to each bacterial experiment, bacterial cultures were refreshed on nutrient agar from stock. Fresh bacterial suspensions were grown overnight at 37 °C in 5 ml of TSB (*E. coli and S. aureus*) or 5 ml YM broth for *C*. *albicans.* Bacterial cells were collected at the logarithmic stage of growth and the suspensions were adjusted to OD₆₀₀ = 0.07.

### Example 5: JIS killing efficacy testing

The tested bacteria were suspended in 5 mL of respective nutrient broth and adjusted to OD₆₀₀ = 0.07. In order to cover the surface, 150 µL of cell suspensions was placed on the surfaces. Experiments were carried out in triplicate at 37 °C. After incubation with the surfaces, the respective cell suspensions were washed and diluted, and each dilution spread on two nutrient agar plates. Resulting colonies were then counted using standard plate counts techniques, and the number of colony forming units per mL was calculated. The number of colony forming units was assumed to be equivalent to the number of viable cells in suspension.

The antibacterial properties against *E. coli* were evaluated for nano-patterned Cu surfaces by using JIS Z 2801:2000 (Japanese Industrial Standard) method. As shown in Fig. 3, all the bacteria were killed after 1 h incubation on Cu(OH)2 nanotubes surface. For the CuO nano-blade surface, 94.5% of *E. coli* bacteria were killed after 1h incubation and all bacteria were killed after 3 hours. In relation to the control, Cu foil with a flat surface, only 28% of bacteria were killed after 1 h and there were still about 35% of *E. coli* surviving after 3 h.

From Fig. 3, it was observed that the *E. coli* killing efficacy was in the order of Cu(OH)₂ nanotubes > CuO nano-blades > Cu foil, which indicated that the sharper the surface, the better the killing efficacy. Considering that the chemical composition of the 3 surfaces are different (Cu(OH)2, CuO, and Cu), to exclude the composition effect, three surfaces were coated with Pt and Cu, respectively, and the E. *coli* killing profiles were re-evaluated.

Fig. 4(A) demonstrates the killing efficacy against *E. coli* for the Pt coated samples. It was shown that Cu foil with Pt coating significantly changed the bacteria killing profile. Without Pt coating, flat Cu foil killed 65% of E. coli after 3 hours (Fig. 3). While after Pt coating, *E. coli* kept on growing instead after 3 hour incubation (Fig. 4). For Cu(OH)₂ nanotubes and CuO nano-blade surface, the killing profiles were almost unchanged after Pt coating as compared with the uncoated surfaces. All the bacteria were killed after 3 hour incubation, as shown in Fig. 4(A). To further confirm this result, three samples were also coated with Cu by vacuum vapour deposition method. SEM results did not show any obvious morphological change after Cu coating. After coating, all the three samples have the same chemical composition of Cu on the nano-patterned surfaces. As shown in Fig. 4(B), the killing profile of Cu-coated flat Cu foil was similar to the uncoated sample shown in Fig. 3. The killing efficacy of Cu(OH)2 nanotubes surface and CuO nano-blades surface were maintained or even increased after coating with Cu. As can be seen from Fig. 4(B), all the bacteria were killed after 1 h incubation with copper coated nanotube and nano-blade surfaces. All these results indicated that the bacteria killing properties of these samples are mainly or entirely contributed by the surface nano-structures rather than chemical component.

The antibacterial activity against *E*. *coli* was also tested for zinc foil and ZnO nanoneedles. As shown in Fig. 5, all the bacteria were killed on ZnO nano-needles surface after 6 h incubation. As control, *E. coli* on flat Zn foil kept on growing, indicating the non-biocidal property of Zn foil. This result again demonstrated that the nano-structured zinc surface kills bacteria efficiently via physical interaction.

In addition to *E. coli,* which represents Gram-negative bacteria, Gram-positive bacteria were also tested. The antibacterial properties against *S. aureus* were also tested, as shown in Fig. 6.

As demonstrated in Fig. 6, the killing profile for *S. aureus* was similar to that of *E. coli.* The Cu(OH)₂ nano-tubes surface and CuO nano-blades surface killed nearly all the bacteria after 1 hour incubation, while for flat Cu foil, 23% of bacteria remained alive even after 3 hours incubation. For ZnO nano-needles surface, all the *S. aureus* were killed after 6 hours incubation, while 70% of *S. aureus* remained surviving on the flat Zn surface.

*C. albicans* as a sample of fungi was also tested. The killing profile for *C. albicans* was very different from those of *E. coli* and *S*. *aureus.* As shown in Fig. 7, all the tested surfaces could kill *C. albicans.* After 24 hours incubation, the remaining *C. albicans* were 2% (Cu), 4% (Cu(OH)₂), 0.7% (CuO), 1.3% (Zn) and 2.8% (ZnO). The nanostructured surfaces did not exhibit faster killing efficacy as compared with the flat surface. This might due to the robust cell wall of fungus as compared with other bacteria. As control, *C*. *albicans* on 6-well plate grow 25 times after 24 hours incubation, indicating the non-antibacterial of plate substrate (results not shown).

### Example 6: Bacterial killing efficacy under washing machine condition

To simulate the washing process, *E. coli* was suspended in 5 ml of water and adjusted to OD₆₀₀ = 0.07. The testing surfaces, mounted on 3.5 cm circular discs, were immersed in 5 ml of 1: 10 diluted bacterial suspension for incubation intervals and shaken at a speed of 300 r/min. The cell suspensions were then sampled (100 µl) at discrete time intervals, serially diluted 1:10, and each dilution spread on two nutrient agar plates. Resulting colonies were then counted, and the number of colony forming units per mL was calculated.

As an example of potential application of the nano-patterned Cu and Zn surfaces in washing machine, the bacterial killing activities of these nano-structured surfaces were tested under a simulated washing machine condition. *E. coli,* water and nanostructured surface were put in a bacterial culture plate, under shaking at 300 r/min. Bacteria in the solution was monitored by plate counting technique. The results show that for Cu(OH)₂ nanotubes and CuO nano-blades surfaces, all the bacteria in water are killed within 30 min. For ZnO nanoneedles surface, 82% of *E*. *coli* was killed after 1 h. In the control experiment, i.e. washing water without the nano-structured surface, the bacteria were still alive after 24 h. This experiment clearly demonstrated the possibility of making the inner surface of a washing machine having antibacterial surface/properties. The surface would kill bacteria during the washing session (30 - 60 min).

In summary, surfaces with Cu(OH)2 nanotubes, CuO nano-blades and ZnO nano-needles have been prepared by simple solution treatment of respective copper or zinc foil at room temperature. All surfaces are bactericidal against *E. coli.* Application of these artificial surfaces are also demonstrated in washing machine condition in water, where *E. coli* bacteria are completely killed within 30 min by Cu(OH)₂ nanotubes and CuO nano-blades surfaces.

### Industrial Applicability

The nano-patterned surfaces of the present application may be useful in providing non-chemical anti-bacteria properties. Such anti-bacterial nano-patterned surfaces may be used as alternative surface materials for frequently-touched surfaces, e.g. doorknobs, handles and sanitary fittings, to provide an environment which discourages or inhibits bacteria proliferation such as in a hospital setting.

Advantageously, this would reduce the reliance on synthetic chemical disinfectants which may undesirably result in secondary contamination and may cause serious drug-resistant superbugs to develop. The disclosed patterned surfaces also lend possibility to the provision of domestic household appliances and equipment possessing such patterned metal surfaces. The anti-bacteria surface may also be used in a number of cleaning applications, e.g. to render the inner chamber surface of household or industrial scale washing machine anti-bacterial. This may advantageously reduce or completely eliminate the requirement of synthetic detergents which may be harmful to the human body. Also, the cleaning time may be reduced which results in higher cleaning efficiency of the washing machine.

## Claims

1. A method of killing or inhibiting the growth of a microorganism, said method comprising the steps of:
providing a zinc substrate having a plurality of, ordered, integrally-formed surface structures prepared by:
contacting a surface of the zinc substrate with a reagent solution, the reagent solution comprising an alkali and a zinc nitrate;
wherein the concentration of the alkali in the reagent solution is from 1.0 M to 2.5 M;
contacting said microorganism with said zinc substrate,
wherein the surface features are micro-sized, nano-sized or a mixture thereof, each surface feature comprising a crystalline phase and at least one pointed terminus.

2. The method of claim 1, wherein the surface structure is a needle, wherein the length of the needle is from 500 nm to 5 µm, wherein the cross-section of the base of the needle is circular having a diameter of 10 nm to 500 nm, and wherein the distal end of the needle is a tip of circular cross-section having a diameter of 1 nm to 100 nm.

3. The method of claim 1 or 2, wherein the concentration of zinc nitrate in the reagent is from 0.01 M to 5 M.

4. The method of claim 3, wherein the concentration of zinc nitrate in the reagent is 0.25M to 0.3 M.

5. The method of any one of the preceding claims, wherein the concentration of the alkali in the reagent solution is 2 M.

6. The method of any one of the preceding claims, wherein the alkali is NaOH or KOH.

7. The method of any one of the preceding claims, wherein the step of contacting the surface of the zinc substrate with the reagent solution is for a duration of from 6 to 18 hours.

8. The method of claim 7, wherein the step of contacting the surface of the zinc substrate with the reagent solution is for a duration of 12 hours.

9. The method of any one of the preceding claims, wherein the step of contacting the surface of the zinc substrate with the reagent solution is at room temperature.

10. The method of claim any one of the preceding claims, wherein the microorganism is bacteria.

11. The method of claim 10, wherein the microorganism is gram-negative or gram-positive bacteria or wherein the microorganism is a gram-negative bacteria selected from the group consisting of Escherichia, Shigella, and Salmonella or wherein the microorganism is a gram-positive bacteria selected from the group consisting of Staphylococcus, Enterococcus and Streptococcus.

12. Use of the zinc substrate as defined in any one of the preceding claims for providing antibacterial properties to an ex-vivo environment.

13. The use of claim 12 for providing bacteriostatic or bactericidal purposes to said ex-vivo environment, is being a non-therapeutic use.

14. The use of any one of claims 12-13, wherein the antibacterial substrate is capable of killing or inhibiting the growth of gram-negative and gram-positive bacteria or wherein the gram-negative bacteria is selected from the group consisting of Escherichia, Shigella, and Salmonella or wherein the gram-positive bacteria is selected from the group consisting of Staphylococcus, Enterococcus and Streptococcus.

## Patentansprüche

1. Ein Verfahren zum Abtöten oder Hemmen des Wachstums eines Mikroorganismus, wobei das Verfahren die Schritte umfasst:
Bereitstellen eines Zinksubstrats mit mehreren geordneten, einstückig ausgebildeten Oberflächenstrukturen, hergestellt durch:
Kontaktieren einer Oberfläche des Zinksubstrats mit einer Reagenzlösung, wobei die Reagenzlösung ein Alkali und ein Zinknitrat umfasst;
wobei die Konzentration des Alkalis in der Reagenzlösung 1,0 M bis 2,5 M beträgt;
Kontaktieren des Mikroorganismus mit dem Zinksubstrat,
wobei die Oberflächenmerkmale mikrogroß, nanogroß oder eine Mischung davon sind,
wobei jedes Oberflächenmerkmal eine kristalline Phase und mindestens ein spitzes Ende umfasst.

2. Das Verfahren nach Anspruch 1, wobei die Oberflächenstruktur eine Nadel ist, wobei die Länge der Nadel 500 nm bis 5 µm beträgt, wobei der Querschnitt des distalen Endes der Nadel kreisförmig ist und einen Durchmesser von 10 nm bis 500 nm aufweist, und wobei das distale Ende der Nadel eine Spitze mit kreisförmigem Querschnitt mit einem Durchmesser von 1 nm bis 100 nm ist.

3. Das Verfahren nach Anspruch 1 oder 2, wobei die Konzentration von Zinknitrat in dem Reagenz 0,01 M bis 5 M beträgt.

4. Das Verfahren nach Anspruch 3, wobei die Konzentration von Zinknitrat in dem Reagenz 0,25 M bis 0,3 M beträgt.

5. Das Verfahren nach einem der vorhergehenden Ansprüche, wobei die Konzentration des Alkalis in der Reagenzlösung 2 M beträgt.

6. Das Verfahren nach einem der vorhergehenden Ansprüche, wobei das Alkali NaOH oder KOH ist.

7. Das Verfahren nach einem der vorangehenden Ansprüche, wobei der Schritt des Kontaktierens der Oberfläche des Zinksubstrats mit der Reagenzlösung für eine Dauer von 6 bis 18 Stunden erfolgt.

8. Das Verfahren nach Anspruch 7, wobei der Schritt des Kontaktierens der Oberfläche des Zinksubstrats mit der Reagenzlösung für eine Dauer von 12 Stunden erfolgt.

9. Das Verfahren nach einem der vorangehenden Ansprüche, wobei der Schritt des Kontaktierens der Oberfläche des Zinksubstrats mit der Reagenzlösung bei Raumtemperatur erfolgt.

10. Das Verfahren nach einem der vorhergehenden Ansprüche, wobei der Mikroorganismus ein Bakterium ist.

11. Das Verfahren nach Anspruch 10, wobei der Mikroorganismus ein gram-negatives oder gram-positives Bakterium ist oder wobei der Mikroorganismus ein gram-negatives Bakterium ist, ausgewählt aus der Gruppe bestehend aus *Escherichia, Shigella* und *Salmonella,* oder wobei der Mikroorganismus ein gram-positives Bakterium ist, ausgewählt aus der Gruppe bestehend aus Staphylococcus, Enterococcus und *Streptococcus.*

12. Verwendung des Zinksubstrats nach einem der vorhergehenden Ansprüche zur Bereitstellung antibakterieller Eigenschaften für eine Ex-vivo-Umgebung.

13. Die Verwendung nach Anspruch 12, zur Bereitstellung bakteriostatischer oder bakterizider Zwecke für die Ex-vivo-Umgebung, die eine nicht-therapeutische Verwendung ist.

14. Die Verwendung nach einem der Ansprüche 12-13, wobei das antibakterielle Substrat in der Lage ist, gram-negative und gram-positive Bakterien oder gram-negative Bakterien ausgewählt aus der Gruppe bestehend aus *Escherichia, Shigella* und *Salmonella* oder gram-positive Bakterien ausgewählt aus der Gruppe bestehend aus *Staphylococcus, Enterococcus* und *Streptococcus,* abzutöten oder deren Wachstum zu hemmen.

## Revendications

1. Procédé de destruction ou d'inhibition de la croissance d'un micro-organisme, ledit procédé comprenant les étapes de :
mise à disposition d'un substrat de zinc possédant une pluralité de structures de surface ordonnées, formées d'un seul tenant préparé par :
mise en contact d'une surface du substrat de zinc avec une solution de réactif, la solution de réactif comprenant un alcali et un nitrate de zinc ;
la concentration de l'alcali dans la solution de réactif étant de 1,0 M à 2,5 M ;
mise en contact dudit micro-organisme avec ledit substrat de zinc,
les caractéristiques de surface étant de taille micrométrique, de taille nanométrique ou
un mélange correspondant, chaque caractéristique de surface comprenant une phase cristalline et au moins une terminaison en pointe.

2. Procédé selon la revendication 1, la structure de surface étant une aiguille, la longueur de l'aiguille étant de 500 nm à 5 µm, la section transversale de la base de l'aiguille étant circulaire possédant un diamètre de 10 nm à 500 nm, et l'extrémité distale de l'aiguille étant une pointe à section transversale circulaire possédant un diamètre de 1 nm à 100 nm.

3. Procédé selon la revendication 1 ou 2, la concentration de nitrate de zinc dans le réactif étant de 0,01 M à 5 M.

4. Procédé selon la revendication 3, la concentration de nitrate de zinc dans le réactif étant de 0,25 M à 0,3 M.

5. Procédé selon l'une quelconque des revendications précédentes, la concentration de l'alcali dans la solution de réactif étant de 2 M.

6. Procédé selon l'une quelconque des revendications précédentes, l'alcali étant NaOH ou KOH.

7. Procédé selon l'une quelconque des revendications précédentes, l'étape de mise en contact de la surface du substrat de zinc avec la solution de réactif étant réalisée pendant une durée allant de 6 à 18 heures.

8. Procédé selon la revendication 7, l'étape de mise en contact de la surface du substrat de zinc avec la solution de réactif étant réalisée pendant une durée de 12 heures.

9. Procédé selon l'une quelconque des revendications précédentes, l'étape de mise en contact de la surface du substrat de zinc avec la solution de réactif étant réalisée à température ambiante.

10. Procédé selon l'une quelconque des revendications précédentes, le micro-organisme étant une bactérie.

11. Procédé selon la revendication 10, le micro-organisme étant des bactéries gram négatives ou gram positives ou le micro-organisme étant une bactérie gram négative choisie dans le groupe constitué par Escherichia, Shigella et Salmonella ou, le micro-organisme étant une bactérie gram positive choisie dans le groupe constitué par Staphylococcus, Enterococcus et Streptococcus.

12. Utilisation du substrat de zinc tel que défini dans l'une quelconque des revendications précédentes pour fournir des propriétés antibactériennes à un environnement *ex vivo.*

13. Utilisation selon la revendication 12 pour fournir des fins bactériostatiques ou bactéricides audit environnement *ex vivo,* qui est une utilisation non thérapeutique.

14. Utilisation selon l'une quelconque des revendications 12 à 13, le substrat antibactérien étant capable de détruire ou d'inhiber la croissance de bactéries gram négatives et gram positives ou, la bactérie gram négative étant choisie dans le groupe constitué par Escherichia, Shigella et Salmonella ou, la bactérie gram positive étant choisie dans le groupe constitué par Staphylococcus, Enterococcus et
